(19) Europäisches Patentamt
European Patent Office
Office européen des brevets

(11) Veröffentlichungsnummer: **0 261 572**
**A1**

(12) **EUROPÄISCHE PATENTANMELDUNG**

(21) Anmeldenummer: **87113529.9**

(22) Anmeldetag: **16.09.87**

(51) Int. Cl.[4]: **C07D 319/12**, C07C 59/10

(30) Priorität: **20.09.86 DE 3632103**
**19.03.87 DE 3708915**

(43) Veröffentlichungstag der Anmeldung:
**30.03.88 Patentblatt 88/13**

(84) Benannte Vertragsstaaten:
**AT BE CH DE ES FR GB GR IT LI LU NL SE**

(71) Anmelder: **BOEHRINGER INGELHEIM KG**

**D-6507 Ingelheim am Rhein(DE)**

(84) **AT BE CH DE ES FR GB GR IT LI LU NL SE**

(71) Anmelder: **Boehringer Ingelheim International G.m.b.H**

**D-6507 Ingelheim am Rhein(DE)**

(84) **GB**

(72) Erfinder: **Müller, Manfred, Dr.-Ing.**
**Alsbacher Strasse 40**
**D-6101 Bickenbach(DE)**

(54) **Verfahren zur Herstellung von Lactid.**

(57) Die Erfindung betrifft ein Verfahren zur Herstellung von Lactid in technischem Maßstab, insbesondere von optisch reinem L(-)-oder D( + )-Lactid.

EP 0 261 572 A1

# Verfahren zur Herstellung von Lactid

Die Erfindung betrifft ein Verfahren zur Herstellung von Lactid, insbesondere von optisch reinem L(-)- bzw. D(+)-Lactid in einem technischen Maßstab.

Die Darstellung von Lactid ist seit längerem aus zahlreichen Patent-und Offenlegungsschriften bekannt.

So beansprucht die deutsche Patentschrift 26 78 26 ein Verfahren zur Darstellung von Lactid, bei dem Milchsäure langsam auf eine Temperatur von 200°C erhitzt und das dabei entstehende Lactid, vorteilhafterweise im Vakuum; abdestilliert wird.

Aus der deutschen Patentschrift 12 34 703 wird die Herstellung von optisch aktivem L(-)-Lactid aus einer wäßrigen Lösung von L(+)-Milchsäure in Gegenwart von Titantetraalkylat offenbart. Die deutsche Auslegeschrift 10 83 275 beschreibt ein Herstellungsverfahren, bei dem die Depolymerisation in Gegenwart von Metallen oder ihrer Verbindungen der IV, V oder VIII Gruppe des Periodensystems erfolgen kann. In den Beispielen sind Zinkoxyd, Zinnoxyd und Antimonoxyd als Katalysatoren erwähnt. Weiterhin ist aus der deutschen Offenlegungsschrift 15 43 958 die Verwendung von Blei(II)-stearat als Depolymerisationskatalysator bekannt.

Die genannten Publikationen beschreiben in ihren Beispielen lediglich Ansätze im Labormaßstab, d.h. bis ca. 1 kg. Wie die Praxis jedoch gelehrt hat, ist bei der Herstellung von Lactid, insbesondere optisch reinem Lactid, die direkte Übertragung auch erfolgreicher Laborversuche in den technischen Maßstab problematisch, wobei als Kriterien u.a. die optische Reinheit und die chemische Ausbeute anzusehen sind. Die in der DE-AS 10 83 275 offenbarten Ausbeuten der beschriebenen Verfahren erwiesen sich bereits in Laborversuchen als nicht reproduzierbar.

Während J. Klein in Makromol. Chem. 30, 35 (1959) berichten, daß die Depolymerisation von Polylactid in Gegenwart von Zinkstaub in 90%iger Ausbeute verläuft, können diese hohen Ausbeuten in einem technisch durchgeführten Produktionsverfahren nicht erreicht werden. Ein technisches Verfahren wie es industriell praktiziert wird, verwendet als Ausgangsmaterial L(+)-Milchsäure, die thermisch in L(-)-Polymilchsäure überführt und anschließend in Gegenwart eines Zink-Pulver-Katalysators zu L(-)-Lactid depolymerisiert wird. Nach diesem Verfahren können bei einem Ansatz ausgehend von ca. 440 kg L(+)-Milchsäure ca. 168 kg optisch aktives L(-)-Lactid in zufriedenstellender Qualität erhalten werden, das entspricht einer Ausbeute von ca. 50 bis 55 % (bezogen auf L(+)-Milchsäure). Bei diesem Verfahren ist es notwendig nach jeder Charge das Reaktionsgefäß zu reinigen, wozu im allgemeinen halbkonzentrierte Natronlauge Verwendung findet.

Abgesehen davon, daß bei dem beschriebenen technischen Verfahren lediglich Ausbeuten zwischen 50 und 55 % erreicht werden können, erwies sich dieses Verfahren als sehr kostenintensiv, da die Reaktionsapparatur sowohl während der Entwässerung der L(+)-Milchsäure wie auch während der Reinigungsphase nicht zur Herstellung von L(-)-Lactid genutzt werden kann.

Versuche, dieses absatzweise (batchweise) arbeitende Verfahren auf ein kontinuierliches oder halbkontinuierliches Verfahren umzustellen, führte überraschenderweise zu einer Verschlechterung der Gesamtausbeute und einer Verminderung der optischen Reinheit des L(-)-Lactids.

Ein weiterer Nachteil der aus dem Stand der Technik bekannten Herstellverfahren ist, daß zur Erzielung einer hohen optischen Reinheit bei optisch aktiven Lactiden an die Depolymerisation mehrere Reinigungsverfahren nachgeschaltet werden müssen.

Es ist Aufgabe der vorliegenden Erfindung, ein Verfahren zur Herstellung von Lactid, insbesondere von optisch reinem L(-) bzw. D(+)-Lactid in technischem Maßstab zur Verfügung zu stellen, welches eine bessere Ausnutzung der eingesetzten Rohstoffe ermöglicht.

Es ist eine weitere Aufgabe der vorliegenden Erfindung ein Verfahren zur Herstellung von optisch aktivem Lactid vorzuschlagen, das sowohl eine höhere chemische Ausbeute wie auch eine höhere optische Ausbeute aufweist.

Erfindungsgemäß wird die Aufgabe dadurch gelöst, daß Polymilchsäure in Gegenwart von 0.05 bis 1.0 Gew.-% Zinnstaub, eines Zinnhalogenids oder einer organischen Zinnverbindung, abgeleitet von einer Carbonsäure mit bis zu 20 Kohlenstoffatomen bei vermindertem Druck auf 130 bis 230°C erhitzt, das sich bildende Lactid abdestilliert und kontinuierlich oder batchweise Polymilchsäure nachgefüllt wird.

Besonders geeignete Zinnverbindungen als Katalysatoren sind Verbindungen der allgemeinen Struktur

$$Sn^{II}(O-\underset{\underset{O}{\|}}{C}-X)_2,$$

worin X einen verzweigten oder unverzweigten Alkyl-, Hydroxyalkyl-oder Alkenylrest mit bis zu 19 Kohlenstoffatomen oder einen Naphthylrest bedeutet, oder Verbindungen der allgemeinen Struktur

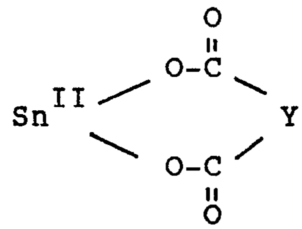

worin Y einen verzweigten oder unverzweigten Alkyl-, Hydroxyalkyl-oder Alkenylrest mit bis zu 18 Kohlenstoffatomen oder einen Phenylrest bedeutet.

Geeignete Alkylgruppen für X,und Y sind beispielsweise der Methyl-, Ethyl-, n-oder iso-Propyl-, n-, sec.- oder tert.-Butyl, Pentyl-, Hexyl-, Heptyl-oder Octylrest, der gegebenenfalls ein oder mehrere Hydroxygruppen enthalten kann. Entsprechende Alkenylreste enthalten eine oder mehrere Doppelbindungen. Wird ein Zinnhalogenid, wie z.B. $SnCl_2$ oder $SnBr_2$ als Katalysator eingesetzt, so bildet sich unter Abspaltung der entsprechenden Säure Zinnlactat.

Bevorzugte Katalysatoren sind Zinnlactat, Zinntartrat, Zinnoxalat, Zinndicaprylat, Zinndilaurat, Zinndipalmitat, Zinndistearat, das Zinndioleat (Derivat der Ölsäure), Zinn-$\alpha$-naphthoeat oder Zinn-$\beta$-naphtoeat. Besonders bevorzugt ist Zinndioctoat, besser bezeichnet als Zinn-di-(2-ethylhexanoat) oder Zinnstaub..

Die optisch aktiven Lactide, L(-)-Lactid und D(+)-Lactid, werden nach analogem Verfahren ausgehend von den entsprechenden L(-)-Polymilchsäuren bzw. D(+)-Polymilchsäuren hergestellt.

Die in das Verfahren eingesetzte Polymilchsäure kann in einem gesonderten Reaktionsschritt nach bekannten Verfahren durch Entwässern von Milchsäure hergestellt werden.

Im Fall von optisch aktiven Polymilchsäuren erfolgt die Herstellung des Ausgangsmaterials selbstverständlich aus den entsprechend optisch aktiven Milchsäuren (L(+)-Milchsäure → L(-)-Polymilchsäure; D(-)-Milchsäure → D(+)-Polymilchsäure).

In einer besonderen Ausführungsform des erfindungsgemäßen Verfahrens wird anstelle der Polymilchsäure Milchsäure eingesetzt. In einem ersten Reaktionsschritt wird die Milchsäure in Gegenwart des Katalysators bei vermindertem Druck bei ansteigender Temperatur entwässert. Im allgemeinen erfolgt die Entwässerung bei Drucken von ca. 0.01 bis 0.05 bar, wobei die Temperaturen im Reaktor auf ca. 150 bis 170°C ansteigt. Wenn ein durchschnittliches Molekulargewicht der dabei gebildeten Polymilchsäure von ca. 400 bis 2000, bevorzugt 600 - 800, erreicht ist, wird kontinuierlich Lactid abdestilliert und anschließend kontinuierlich oder batchweise Polymilchsäure nachgefüllt.

In einer weiteren Ausführungsform kann in das kontinuerliche Verfahren anstelle von Polymilchsäure auch Milchsäure nachgezogen werden.

Im nachfolgenden wird das Verfahren zur Herstellung von L(-))-Lactid näher beschrieben, dieses ist jedoch ebenso auf die Herstellung von D(+)-Lactid, D,L-Lactid und meso-Lactid, durch den Einsatz der entsprechenden Polymilchsäuren, bzw. Milchsäuren übertragbar.

Beim Anfahren des Reaktors wird L(-)-Polymilchsäure vorgelegt und mit 0.05 bis 1.0 Gew.-%, bevorzugt 0.1 bis 0.8 Gew.-% Zinnstaub, Zinnhalogenid oder der zinnorganischen Verbindung versetzt. Anschließend wird bei vermindertem Druck auf 130 bis 230°C, bevorzugt 180 bis 200°C erhitzt, wobei das entstehende L(-)-Lactid abdestilliert wird. Der optimale Temperaturbereich ist von dem angelegten Vakuum abhängig und kann durch einfache Versuche ermittelt werden. Eine möglichst niedrige Destillationstemperatur wirkt sich günstig auf die Reinheit des Destillats aus. Nachdem eine bestimmte Produktmenge abdestilliert ist, wird L(-)-Polymilchsäure nachgefüllt. Dies geschieht zweckmäßigerweise in geschmolzener Form. Das Nachfüllen kann batchweise (portionsweise) oder aber auch in kontinuierlicher Form, z.B. durch Zutropfen, geschehen. Dabei kann die nachgefüllte Menge durchaus größer sein als die ursprüngliche, beim Start der Reaktion eingesetzte Menge.

Für den Fall, daß die L(-)-Polymilchsäure batchweise nachgefüllt wird, ist das Restvolumen des Reaktorinhalts in einem weiten Bereich bezüglich der Qualität des Produktes unkritisch, es ist jedoch zweckmäßig, nach ca. 50 bis 90%igem Umsatz aufzufüllen. Es ist nicht auszuschließen, daß ein zu weites Absenken des Reaktorinhalts zu einer Produktverschlechterung führt. Im Fall der kontinuierlichen Produktführung erfolgt der Zulauf zweckmäßiger so, daß das Volumen des Reaktorinhalts weitestgehend konstant gehalten wird.

Wird anstelle der L(-)-Polymilchsäure direkt L(+)-Milchsäure in den Reaktor eingesetzt, erfolgt vor der Depolymerisation zu Lactid in Gegenwart des Katalysators der zinnorganischen Verbindung oder des Zinnstaubs eine Entwässerung der L(+)-Milchsäure zu L(-)-Polymilchsäue bis zu einem mittleren Molekulargewicht von ca. 400 - 2000, bevorzugt 500 bis 800. Die Entwässerung erfolgt vorzugsweise bei ca. 0.03 bar bei ansteigender Temperatur bis ca. 170°C. Nachdem das gewünschte Molekulargewicht erreicht ist, wird der Ansatz wie zuvor beschrieben weiterverarbeitet.

Wie bereits ausgeführt, kann in einer weiteren Verfahrensvariante anstelle von L(-)-Polymilchsäure auch L(+)-Milchsäure nachgezogen werden, wobei dann zuerst wider Wasser aus dem Reaktionsgemisch bis zum Erhalt des gewünschten Molekulargewichtes der Polymilchsäure abdestilliert wird. Das weitere Verfahren erfolgt dann wie oben beschrieben.

Der Einsatz bzw. das Nachfüllen von L(+)-Milchsäure anstelle von L(-)-Polymilchsäure hat gegenüber den zuvor beschriebenen Varianten keinerlei Nachteile bezüglich chemischer und optischer Ausbeute. Von Vorteil ist, daß die Reaktionszeit zur Entwässerung der Milchsäure um ca. 50 % verkürzt wird. Das Molekulargewicht der entstehenden Polymilchsäure wird durch Titration der Endgruppen bestimmt.

Das abdestillierte L(-)-Lactid wird nach bekannten Verfahren aufgearbeitet, z.B. durch Umkristallisieren in Alkohol mit 1 bis 6, bevorzugt 1 - 3, Kohlenstoffatomen, besonders bevorzugt Isopropanol, oder durch Lösen und anschließendem Fällen in einem Nichtlösemittel.

Nach dem erfindungsgemäßen Verfahren erhält man nach der Aufarbeitung einschließlich Umkristallisation optisch reines L(-)-Lactid in ca. 80 %iger Ausbeute, bezogen auf den Einsatz von L(+)-Milchsäure.

Vorteile des erfindungsgemäßen Verfahrens sind eine hohe Ausbeute bei einem im technischen Maßstab durchgeführten Herstellungsverfahren bei hoher optischer Reinheit, eine bessere Ausnutzung des eigentlichen Depolymerisationsreaktors, so daß auch mit realtiv kleinen Anlagen ein hoher Durchsatz (gemessen in kg/h) zu erreichen ist.

Während nach dem herkömmlichen Prozeß, unter Verwendung von Zinkstaub, gegen Ende der Reaktion eine deutliche Qualitätseinbuße des abdestillierten L(-)-Lactids beobachtet wurde, erhält man nach dem erfindungsgemäßen Verfahren auch nach wiederholtem Nachziehen an L(-)-Polymilchsäure oder L(+)-Milchsäure eine gleichbleibende hohe Qualität; entsprechendes gilt auch für die anderen Lactide.

Das erfindungsgemäße Verfahren ermöglicht die Herstellung von optisch aktiven Lactiden, insbesondere von L(-)-Lactid mit einer optischen Reinheit von > 99% ee dadurch, daß im Anschluß an die Destillation eine einzige Reinigungsoperation, wie z.B. Umkristallisieren, angeschlossen wird. Bevorzugt ist die Umkristallisation aus Isopropanol.

Die nachfolgenden Beispiele erläutern die Erfindung, jedoch ohne sie zu beschränken

Vergleichsbeispiel: Herkömmliches Verfahren unter Verwendung von Zink als Katalysator.

440 kg L(+)-Milchsäure (90 %-ig) werden mit 2,2 kg Zinkstaub versetzt und bis ca. 180°C Sumpftemperatur und ca. 0,035 bar entwässert. Anschließend wird bis ca. 230°C und ca. 0,01 bar das entstehende L(-)-Lactid abdestilliert. Man erhält ca. 263 kg L(-)-Lactid als Destillat. Der Rückstand, ca. 10 - 20 kg, wird mittels ca. 150 kg verdünnter Natronlauge entfernt. Das erhaltene Destillat wird in 263 kg Isopropanol eingebracht, wobei man 195 kg L(-)-Lactid als Rohkristalle erhält. Durch Umkristallisieren aus Isopropanol unter Zusatz von Aktivkohle werden 168 kg L(-)-Lactid, = 53 % bez. auf L(+) Milchsäure, mit einem Schmp. von 96 - 98°C und $[\alpha]_D^{25}$ > -287° erhalten.

<u>Beispiel 1</u>

232 kg L(-)-Polymilchsäure mit einem durchschnittlichen Molekulargewicht von 610 werden mit 1 kg Zinnstaub versetzt und auf 194°C - 198°C, bei einem Vakuum von 25-13 Torr, erhitzt. Hierbei destilliert L(-)-Lactid ab. Nach einer Destillatmenge von 190 kg L(-)-Lactid werden 220 kg L(-)-Polymilchsäure nachgezogen und unter vorgenannten Bedingungen destilliert.

Dieses Verfahren widerholt sich mehrmals:

| Destillatmenge: | | Nachgezogen: | |
|---|---|---|---|
| | 188 kg | | 186 kg |
| | 154 kg | | 226 kg |
| | 178 kg | | 405 kg |
| | 355 kg | | 393 kg |
| | 370 kg | | 396 kg |
| | 363 kg | | 409 kg |
| | 378 kg | | 429 kg |
| | 398 kg | | 401 kg |
| | 374 kg | | |

Rückstand : 20 kg
Durchsatz : L(-)-Polymilchsäure: ca. 44 kg/h

Das Destillat wird wie im Vergleichsbeispiel beschrieben, aufgearbeitet.

Gesamtausbeute: 2110 kg L(-)-Lactid = 64,8 % d.Th. b.a. Einsatz L(+)-Milchsäure
optische Reinheit: > 99 % e e L(-)-Lactid
Spezifikation: 96 - 98°C
$[a]_D^{25}$ > -289,6° (Toluol)

Anstelle von Sn-Staub oder Sn-Pulver kann die Reaktion auch mit 3,4 kg Zinndioctoat durchgeführt werden. Der Durchsatz erhöht sich dabei auf 75 kg/h.

Beispiel 2

798 g L(+)-Milchsäure 90 %-ig (Lactol 90) werden mit 3,4 g Zinndioctoat versetzt und bei ca. 170°C Sumpftemperatur zu Polymilchsäure umgesetzt. Nach 4,0 h ist ein durchschnittliches Molekulargewicht von 748 erreicht. Danach wird bei ca. 0,01 bar und bis ca. 200°C Sumpftemperatur in 1 h 35' 531 g L(-)-Lactid abdestilliert. Auf den Destillationssumpf wird Lactol 90 nachgezogen und unter vorgenannten Bedingungen wiederum zu L(-)-Lactid umgesetzt. Dieses Verfahren widerholt sich mehrmals:

| Nachgezogen Lactol 90 g | Bildung PMS Zeit | MG | Destillation L(-)-Lactid Zeit | Menge Destillat g |
|---|---|---|---|---|
| 800 | 3 h 50' | 724 | 2 h 30' | 561 |
| 795 | 4 h | 687 | 2 h 25' | 529 |
| 795 | 3 h 40' | 778 | 2 h 10' | 542 |
| 827 | 9 h 35' | 1380 | 2 h | 568 |

Rückstand: 30,0 g
Veresterungszeit bis MG von ca. 750 : 3 h 50'
Durchsatz Destillat: 242 g/h

Das L(-)-Lactid wird nach bekanntem Verfahren aufgearbeitet.

Gesamtausbeute: 2000,4 g = 69,4 % d.Th. b.a. Einsatz L(+)-Milchsäure.

Beispiel 3

2 g Zinn werden in 606 g Lactol 90 gelöst. Die Lösung wird bei ca. 0,02 bar unter Abdestillieren von Wasser bis ca. 170°C zu Polymilchsäure umgesetzt. Nach 3,0 h ist ein durchschnittliches Molekulargewicht von 834 erreicht. Danach wird bei ca. 0,01 bar und bis ca. 215°C Sumpftemperatur in 35' 360 g Destillat abdestilliert. Auf den Destillationssumpf werden 723 g Polymilchsäure mit einem durchschnittlichen Molekulargewicht von 570 nachgezogen und unter oben genannten Bedingungen zu L(-)-Lactid umgesetzt. Man erhält 711 g Destillat in siebzig Minuten.

Rückstand: 30 g

Durchsatz Destillat: 612 g/h

Das L(-)-Lactid wid nach bekanntem Verfahren aufgearbeitet.

Gesamtausbeute: 797,2 g = 69,5 % d.Th. b.a. Einsatz L(+)-Milchsäure

Die in den Beispielen 2 und 3 beschriebenen Laborverfahren sind ohne weiteres in technischem Maßstab übertragbar.

Beispiel 4

662 g L(-)-Polymilchsäure mit einem durchschnittlichen Molekulargewicht von 1080 werden mit 3,4 g Zinndioctoat versetzt und bei ca. 200°C Sumpftemperatur und einem Druck von ca. 0,01 bar in 1,5 Stunden 643 g L(-)-Lactid abdestilliert. Auf den Destillationssumpf wird Polymilchsäure nachgezogen und unter vorgenannten Bedingungen widerum zu L(-)-Lactid umgesetzt. Dieses Verfahren widerholt sich mehrmals:

| Nachgezogen Polymilchsäure g | MG | Destillation L(-)-Lactid Zeit | Menge Destillat g |
|---|---|---|---|
| 700 | 1080 | 1 h 12' | 702 |
| 805 | 1080 | 1 h 25' | 791 |

Rückstand: 20,0 g

Durchsatz Destillat: 585 g/h

Das L(-)-Lactid wird nach bekanntem Verfahren aufgearbeitet.

Gesamtausbeute:1779 g = 79,6 % d.Th. b.a. Einsatz L(+)-Milchsäure.

**Ansprüche**

1) Verfahren zur Herstellung von Lactid in technischem Maßstab, dadurch gekennzeichnet, daß Polymilchsäure in Gegenwart von 0.05 bis 1.0 Gew.-% Zinnstaub, ein Zinnhalogenid oder einer organischen Zinnverbindung, abgeleitet von einer Carbonsäure mit bis zu 20 Kohlenstoffatomen, bei vermindertem Druck auf 130 bis 230°C erhitzt, das sich bildende Lactid abdestilliert und kontinuierlich oder batchweise Polymilchsäure nachgefüllt wird.

2) Verfahren zur Herstellung von optisch reinem L(-)-Lactid in technischem Maßstab, dadurch gekennzeichnet, daß L(-)-Polymilchsäure in Gegenwart von 0.05 bis 1.0 Gew.-% Zinnstaub, Zinnhalogenid oder einer organischen Zinnverbindung, abgeleitet von einer Carbonsäure mit bis zu 20 Kohlenstoffatomen, bei vermindertem Druck auf 130 bis 230°C erhitzt, das sich bildende L(-)-Lactid abdestilliert und kontinuierlich oder batchweise L(-)-Polymilchsäure nachgefüllt wird.

3) Verfahren zur Herstellung von optisch reinem D(+)-Lactid in technischem Maßstab, dadurch gekennzeichnet, daß D(+)-Polymilchsäure in Gegenwart von 0.05 bis 1.0 Gew.-% Zinnstaub, Zinnhalogenid oder einer organischen Zinnverbindung, abgeleitet von einer Carbonsäure mit bis 20 Kohlenstoffatomen bei vermindertem Druck auf 130 bis 230°C erhitzt, das sich bildende D(+)-Lactid abdestilliert und kontinuierlich oder batchweise D(+)-Polymilchsäure nachgefüllt wird.

4) Verfahren zur Herstellung von Lactid in technischem Maßstab, dadurch gekennzeichnet, daß Milchsäure in Gegenwart von 0.05 bis 1.0 Gew.-% Zinnstaub, Zinnhalogenid oder einer organischen Zinnverbindung, abgeleitet von einer Carbonsäure mit bis zu 20 Kohlenstoffatomen, bei vermindertem Druck und ansteigender Temperatur entwässert und in Polymilchsäure mittleren Molekulargewichts überführt und anschließend bei vermindertem Druck bei 130 bis 230°C das sich bildende Lactid abdestilliert und kontinuierlich oder batchweise Polymilchsäure nachgefüllt wird.

5) Verfahren nach Anspruch 4, dadurch gekennzeichnet, daß das gebildete Lactid abdestilliert wird und batchweise Milchsäure nachgefüllt wird.

6) Verfahren zur Herstellung von optisch reinem L(-)-oder D(+)-Lactid nach Anspruch 4 oder 5, dadurch gekennzeichnet, daß man die optisch aktiven Milchsäuren bzw. Polymilchsäuren in das Verfahren einsetzt.

7) Verfahren zur Herstellung von Polymilchsäure mit einem mittleren Molekulargewicht von 400 bis 2000, dadurch gekennzeichnet, daß man Milchsäure in Gegenwart von Zinnstaub, Zinnhalogenid oder einer organischen Zinnverbindung unter vermindertem Druck auf ca. 150 bis 170°C erhitzt.

8) Verfahren nach einem der Ansprüche 1 bis 7, dadurch gekennzeichnet, daß die organische Zinnverbindung die allgemeine Struktur $Sn^{II}(O-\underset{\underset{O}{\|}}{C}-X)_2$

aufweist,

worin X einen verzweigten oder unverzweigten Alkyl-Hydroxyalkyl-oder Alkenylrest mit bis zu 19 Kohlenstoffatomen oder einen Naphthylrest bedeutet oder die allgemeine Struktur

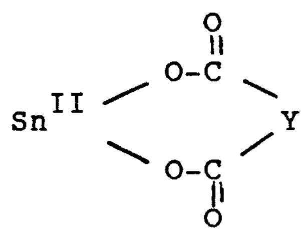

aufweist, worin Y einen verzweigten oder unverzweigten Alkyl-, Hydroxyalkyl-oder Alkenylrest mit bis zu 18 Kohlenstoffatomen oder einen Phenylrest bedeutet.

9) Verfahren nach einem der Ansprüche 1 bis 7, dadurch gekennzeichnet, daß die organische Zinnverbindung aus der Gruppe Zinnlactat, Zinntartrat, Zinnoxalat, Zinndicaprylat, Zinndilaurat, Zinndipalmitat, Zinndistearat, Zinndioleat, Zinn-α-naphthoeat, Zinn-β-napthoeat und Zinndioctoat ausgewählt ist.

Europäisches Patentamt

# EUROPÄISCHER RECHERCHENBERICHT

Nummer der Anmeldung

EP 87113529.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | DE - B1 - 1 083 275 (DYNAMIT NOBEL)<br>* Gesamt *<br>-- | 1-7 | C 07 D 319/12<br>C 07 C 59/10 |
| A | DE - A - 53 074 (ANDRATSCHKE et al.)<br>* Ansprüche 1,2 *<br>-- | 1,4,7 | |
| P,A | CHEMICAL ABSTRACTS, Band 105, Nr. 12, 22. September 1986, Columbus, Ohio, USA<br>COWSAR D.R.; TICE, TH.R.; GILLEY, R.M.; ENGLISH, J.P. "Poly(lactide-co-glycolide)microcapsules for controlled release of steroids" Seite 329, Spalte 2, Zusammenfassung-Nr. 102505x<br>& Methods Enzymol. 1985, 112 (Drug Enzyme Targeting, Pt.A), 101-16<br>-- | 1,4,7 | **RECHERCHIERTE SACHGEBIETE (Int. Cl.4)**<br>C 07 D 319/00<br>C 07 C 59/00 |
| A | CHEMICAL ABSTRACTS, Band 104, Nr. 26, 30. Juni 1986, Columbus, Ohio, USA<br>YOU, Y.; LU, G.; SHEN, Y.; YU, Y.; HE, B.; "Synthesis, application and degredation of polylactic acid" Seite 349, Spalte 1, Zusammenfassung-Nr. 230 312y<br>& Zhongguo Shengwu Yixue Gongcheng Suebao 1985, 4(4), 244-8<br>-- | 1,4,7 | |

Der vorliegende Recherchenbericht wurde für alle Patentansprüche erstellt.

| Recherchenort | Abschlußdatum der Recherche | Prüfer |
|---|---|---|
| WIEN | 16-12-1987 | BRUS |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung



EP 87113529.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 103, Nr. 22, 2. Dezember 1985, Columbus, Ohio, USA<br>RAK. J.; FORD, J.L.; ROSTRON, CH.; WALTERS, V. "The preparation and characterization of poly (DL-lactic acid) for use as a biogradable drug carrier"<br>Seite 357, Spalte 1, Zusammenfassung-Nr. 183 539v<br>& Pharm. Acta Helv. 1985, 60(5-6), 162-9<br>---- | 1,4,7 | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 16-12-1987 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82